# EUROPEAN PATENT APPLICATION

(11) **EP 1 415 665 A2**
(43) Date of publication of application: **06.05.2004**
(21) Application number: 02079559.7
(22) Date of filing: 01.11.2002
(51) Int. Cl.: A61K 39/245, C07K 14/005

(54) **BHV5 mutants**

(71) Applicant: ID-Lelystad, Instituut voor Dierhouderij en Diergezondheid B.V., 8219 PH Lelystad (NL)
(72) Inventor: Rijsewijk, Franciscus Antonius Maria, 8244 AK Lelystad (NL)
(74) Representative: Prins, Adrianus Willem, Mr. Ir.

(57) **Abstract**

The invention relates to the field of encephalitis in ruminants. The invention provides a mutant of bovine herpesvirus 5 (BHV5) provided with a functional deletion in each of at least two genes related to the clinical manifestation of neurovirulence upon infection of a host with BHV5. Furthermore, the invention provides a vaccine comprising such a mutant.

The invention further provides a method for distinguishing a ruminant infected with a wild-type BHV5 strain or vaccinated against BHV5 from within a population of ruminants vaccinated with a vaccine according to the invention comprising testing a ruminant for the presence or absence of an antibody directed against the glycoprotein I (gI) gene or the glycoprotein E (gE) gene or both.

SEQUENCE LISTING

<110> ID-Lelystad, Instituut voor Dierhouderij en Diergezondheid B.V.

<120> BHV5 mutants

<130> P61753EP00

<140> EP 0207559.7

<141> 2002-11-01

<160> 3

<170> PatentIn version 3.1

<210> 1

<211> 9

<212> DNA

<213> Artificial Sequence

<220>

<223> consensus Kozak sequence

<400> 1
gccgccacc 9

<210> 2

<211> 1598

<212> DNA

<213> Artificial Sequence

<220>

<223> synthetic DNA fragment RGRESM which codes for the G protein found
in the "GUY1-BV" rabies

<220>

<221> CDS

<222> (17)..(1591)

<223>

<400> 2
aaggcctgcc gccacc atg atc ctt caa gcc ctt ctc ttt gtc cct ttc ctg 52
Met Ile Leu Gln Ala Leu Leu Phe Val Pro Phe Leu
1               5                   10

atc tct tcc ctc tgc ctc ggg aaa ttc ccc atc tac aca ata ccc gac 100
Ile Ser Ser Leu Cys Leu Gly Lys Phe Pro Ile Tyr Thr Ile Pro Asp
        15                  20                  25

aaa ctc ggc cct tgg agc ccc atc gat ata cat cac ctc tcc tgc cca 148
Lys Leu Gly Pro Trp Ser Pro Ile Asp Ile His His Leu Ser Cys Pro
    30                  35                  40

aat aat ttg gtc gtc gaa gac gaa gga tgc acc agt cta tct gga ttc 196
Asn Asn Leu Val Val Glu Asp Glu Gly Cys Thr Ser Leu Ser Gly Phe
45                  50                  55                  60

tct tac atg gaa cta aaa gtc gga tac atc tct gcc ata aaa gtc aac 244
Ser Tyr Met Glu Leu Lys Val Gly Tyr Ile Ser Ala Ile Lys Val Asn
                65                  70                  75

ggc ttc act tgt acc ggc gtc gtc aca gaa gct gaa acc tat acc aac 292
Gly Phe Thr Cys Thr Gly Val Val Thr Glu Ala Glu Thr Tyr Thr Asn
            80                  85                  90

ttt gtc ggc tat gtc aca ccc aca ttc aag cgc aaa cat ttc cgc cct 340
Phe Val Gly Tyr Val Thr Pro Thr Phe Lys Arg Lys His Phe Arg Pro
        95                  100                 105

ata ccg gat gca tgc cgc gct gca tac aac tgg aaa atg gca ggc gat 388
Ile Pro Asp Ala Cys Arg Ala Ala Tyr Asn Trp Lys Met Ala Gly Asp
    110                 115                 120

ccc cga tat gaa gaa tct ctc caa aat cct tat cct gat tac cac tgg 436
Pro Arg Tyr Glu Glu Ser Leu Gln Asn Pro Tyr Pro Asp Tyr His Trp
125                 130                 135                 140

cta cgg acc gtc aaa acc act aaa gaa tct ctt atc atc ata tct ccg 484
Leu Arg Thr Val Lys Thr Thr Lys Glu Ser Leu Ile Ile Ile Ser Pro
                145                 150                 155

tct gtc gct gat cta gac cca tat gac aaa tcc ctt cat tct cgc gtc 532
Ser Val Ala Asp Leu Asp Pro Tyr Asp Lys Ser Leu His Ser Arg Val
            160                 165                 170

ttc cct ggc ggg aaa tgc ttg gga ata aca gtc tcc tcc acc tac tgc 580
Phe Pro Gly Gly Lys Cys Leu Gly Ile Thr Val Ser Ser Thr Tyr Cys
        175                 180                 185

tca acc aac cat gac tac acc atc tgg atg ccc gaa gaa cca cga ctc 628
Ser Thr Asn His Asp Tyr Thr Ile Trp Met Pro Glu Glu Pro Arg Leu
    190                 195                 200

ggg aca tct tgc gac att ttt acc tca tca aaa ggg aaa aaa gca tct 676
Gly Thr Ser Cys Asp Ile Phe Thr Ser Ser Lys Gly Lys Lys Ala Ser
205                 210                 215                 220

aaa ggc ggc aaa act tgc gga ttc gtc gat gaa cgc ggc ctc tac aaa 724
Lys Gly Gly Lys Thr Cys Gly Phe Val Asp Glu Arg Gly Leu Tyr Lys
                225                 230                 235

agt cta aaa gga gca tgc aaa ctc aaa ctc tgc gga gtc ctc gga ctt 772
Ser Leu Lys Gly Ala Cys Lys Leu Lys Leu Cys Gly Val Leu Gly Leu

            240                 245                 250

aga ctt atg gat gga acc tgg gtc tca att caa aca tcc gac gat acc 820
Arg Leu Met Asp Gly Thr Trp Val Ser Ile Gln Thr Ser Asp Asp Thr
        255                 260                 265

aaa tgg tgc cct ccg gat caa ctt gtc aat cta cat gac ttt cac tca 868
Lys Trp Cys Pro Pro Asp Gln Leu Val Asn Leu His Asp Phe His Ser
    270                 275                 280

gac gaa atc gaa cat ctc gtc gtc aaa gaa ttg gcg aaa aaa cgc gaa 916
Asp Glu Ile Glu His Leu Val Val Lys Glu Leu Ala Lys Lys Arg Glu
285                 290                 295                 300

gaa tgc ttg gat gca ttg gaa tcc atc atg acc acc aaa tcc gtc tct 964
Glu Cys Leu Asp Ala Leu Glu Ser Ile Met Thr Thr Lys Ser Val Ser
                305                 310                 315

ttt cga cga ctc tcc tat ttg cga aaa cta gtc ccc gga ttt gga aaa 1012
Phe Arg Arg Leu Ser Tyr Leu Arg Lys Leu Val Pro Gly Phe Gly Lys
            320                 325                 330

gca tac acc ata ttc aac aaa act ttg atg gag gct gac gcc cac tac 1060
Ala Tyr Thr Ile Phe Asn Lys Thr Leu Met Glu Ala Asp Ala His Tyr
        335                 340                 345

aaa tcc gtc gaa act tgg aac gaa atc atc ccc tca aaa gga tgc tta 1108
Lys Ser Val Glu Thr Trp Asn Glu Ile Ile Pro Ser Lys Gly Cys Leu
    350                 355                 360

aaa gtc cgc gaa cga tgc cat cct cca gtc gac ggc gtc ttc ttc aac 1156
Lys Val Arg Glu Arg Cys His Pro Pro Val Asp Gly Val Phe Phe Asn
365                 370                 375                 380

ggc ata att ctc gga cct gac ggg aat gtc ctg ata cca gaa atg caa 1204
Gly Ile Ile Leu Gly Pro Asp Gly Asn Val Leu Ile Pro Glu Met Gln
                385                 390                 395

tcc tct cta ctt caa caa cat atg gaa ctc ttg gaa tct tct gtg atc 1252
Ser Ser Leu Leu Gln Gln His Met Glu Leu Leu Glu Ser Ser Val Ile
            400                 405                 410

ccc cta atg cat ccc ttg gcg gac ccg tca aca gtc ttc aaa gaa ggg 1300
Pro Leu Met His Pro Leu Ala Asp Pro Ser Thr Val Phe Lys Glu Gly
        415                 420                 425

gat gaa gcg gaa gat ttt gtc gaa gtt cac ctc ccg gat gtc cac aaa 1348
Asp Glu Ala Glu Asp Phe Val Glu Val His Leu Pro Asp Val His Lys
    430                 435                 440

caa atc tcc ggc gtc gac ctt ggc ttc ccg tct tgg ggg aaa tat ctc 1396
Gln Ile Ser Gly Val Asp Leu Gly Phe Pro Ser Trp Gly Lys Tyr Leu
445                 450                 455                 460

ctg atg att gcc ggc ggc ttg gcg aca ctc gtt ctg ata atc tgc tcg 1444
Leu Met Ile Ala Gly Gly Leu Ala Thr Leu Val Leu Ile Ile Cys Ser
                465                 470                 475

atg gca tgc tgc aga aga acc aag cga aca gaa tca cgc cgg cgc ggc 1492
Met Ala Cys Cys Arg Arg Thr Lys Arg Thr Glu Ser Arg Arg Arg Gly
            480                 485                 490

tct ggc gaa tcc gaa aaa aaa gtc acg gca acc ccc caa act cgc aaa 1540
Ser Gly Glu Ser Glu Lys Lys Val Thr Ala Thr Pro Gln Thr Arg Lys
        495                 500                 505

gtc gta tct tca tgg gaa tta tac aaa tca gaa ggc gat gcc cgc ctc 1588
Val Val Ser Ser Trp Glu Leu Tyr Lys Ser Glu Gly Asp Ala Arg Leu

510                 515                 520

tga aggcctt                                                          1598

<210>  3

<211>  524

<212>  PRT

<213>  Artificial Sequence

<220>

<223>  protein coding strand

<400>  3

Met Ile Leu Gln Ala Leu Leu Phe Val Pro Phe Leu Ile Ser Ser Leu
1               5                   10                  15

Cys Leu Gly Lys Phe Pro Ile Tyr Thr Ile Pro Asp Lys Leu Gly Pro
            20                  25                  30

Trp Ser Pro Ile Asp Ile His His Leu Ser Cys Pro Asn Asn Leu Val
        35                  40                  45

Val Glu Asp Glu Gly Cys Thr Ser Leu Ser Gly Phe Ser Tyr Met Glu
    50                  55                  60

Leu Lys Val Gly Tyr Ile Ser Ala Ile Lys Val Asn Gly Phe Thr Cys
65                  70                  75                  80

Thr Gly Val Val Thr Glu Ala Glu Thr Tyr Thr Asn Phe Val Gly Tyr
                85                  90                  95

Val Thr Pro Thr Phe Lys Arg Lys His Phe Arg Pro Ile Pro Asp Ala
            100                 105                 110

Cys Arg Ala Ala Tyr Asn Trp Lys Met Ala Gly Asp Pro Arg Tyr Glu
        115                 120                 125

Glu Ser Leu Gln Asn Pro Tyr Pro Asp Tyr His Trp Leu Arg Thr Val
    130                 135                 140

Lys Thr Thr Lys Glu Ser Leu Ile Ile Ile Ser Pro Ser Val Ala Asp
145                 150                 155                 160

Leu Asp Pro Tyr Asp Lys Ser Leu His Ser Arg Val Phe Pro Gly Gly
                165                 170                 175

Lys Cys Leu Gly Ile Thr Val Ser Ser Thr Tyr Cys Ser Thr Asn His
            180                 185                 190

Asp Tyr Thr Ile Trp Met Pro Glu Glu Pro Arg Leu Gly Thr Ser Cys
        195                 200                 205

Asp Ile Phe Thr Ser Ser Lys Gly Lys Lys Ala Ser Lys Gly Gly Lys
    210                 215                 220

Thr Cys Gly Phe Val Asp Glu Arg Gly Leu Tyr Lys Ser Leu Lys Gly
225                 230                 235                 240

Ala Cys Lys Leu Lys Leu Cys Gly Val Leu Gly Leu Arg Leu Met Asp
                245                 250                 255

Gly Thr Trp Val Ser Ile Gln Thr Ser Asp Asp Thr Lys Trp Cys Pro
            260                 265                 270

Pro Asp Gln Leu Val Asn Leu His Asp Phe His Ser Asp Glu Ile Glu
        275                 280                 285

His Leu Val Val Lys Glu Leu Ala Lys Lys Arg Glu Glu Cys Leu Asp
    290                 295                 300

Ala Leu Glu Ser Ile Met Thr Thr Lys Ser Val Ser Phe Arg Arg Leu
305                 310                 315                 320

Ser Tyr Leu Arg Lys Leu Val Pro Gly Phe Gly Lys Ala Tyr Thr Ile
                325                 330                 335

Phe Asn Lys Thr Leu Met Glu Ala Asp Ala His Tyr Lys Ser Val Glu
            340                 345                 350

Thr Trp Asn Glu Ile Ile Pro Ser Lys Gly Cys Leu Lys Val Arg Glu
        355                 360                 365

Arg Cys His Pro Pro Val Asp Gly Val Phe Phe Asn Gly Ile Ile Leu
    370                 375                 380

Gly Pro Asp Gly Asn Val Leu Ile Pro Glu Met Gln Ser Ser Leu Leu
385                 390                 395                 400

Gln Gln His Met Glu Leu Leu Glu Ser Ser Val Ile Pro Leu Met His
                405                 410                 415

Pro Leu Ala Asp Pro Ser Thr Val Phe Lys Glu Gly Asp Glu Ala Glu
            420                 425                 430

Asp Phe Val Glu Val His Leu Pro Asp Val His Lys Gln Ile Ser Gly
        435                 440                 445

Val Asp Leu Gly Phe Pro Ser Trp Gly Lys Tyr Leu Leu Met Ile Ala
    450                 455                 460

Gly Gly Leu Ala Thr Leu Val Leu Ile Ile Cys Ser Met Ala Cys Cys
465                 470                 475                 480

Arg Arg Thr Lys Arg Thr Glu Ser Arg Arg Arg Gly Ser Gly Glu Ser
                485                 490                 495

Glu Lys Lys Val Thr Ala Thr Pro Gln Thr Arg Lys Val Val Ser Ser
            500                 505                 510

Trp Glu Leu Tyr Lys Ser Glu Gly Asp Ala Arg Leu
        515                 520

## Description

The invention relates to the field of encephalitis in ruminants.

Bovine herpesvirus 5 (BHV5), also known as bovine encephalitis herpesvirus, is one of the etiological agents of fatal encephalitis in cattle and other ruminants. BHV5 is a member of the alphaherpesviruses and has a double stranded DNA genome of about 140 kilo base pairs of which further sequence information is scantily known. BHV5 is found in cattle mainly in the Southern Hemisphere e.g. in Australia, Brazil and Argentina, but sporadic cases in North America and Europe have also been reported. At present no safe and efficacious BHV5 vaccine is available. Cross-protection by a BHV1 vaccine has been reported for a less virulent BHV5 strain, but is found to be insufficient to confer protection against more virulent BHV5 strains. Several genes are referred to as possibly being responsible for the neurovirulence of BHV5 in experimental animals. E.g. the glycoprotein E gene [Chowdhury et al. (2000) Bovine herpesvirus 5 glycoprotein E is important for neuroinvasiveness and neurovirulence in the olfactory pathway of the rabbit. J Virol. 74:2094-2106]; the US9 gene [Chowdhury et al. (2002) Bovine herpesvirus 5 (BHV-5) Us9 is essential for BHV-5 neuropathogenesis. J Virol. 76:3839-3851] and the glycoprotein C gene [Chowdhury et al. (2000) Neurovirulence of glycoprotein C (gC)-deleted bovine herpesvirus type-5 (BHV-5) and BHV-5 expressing BHV-1 gC in a rabbit seizure model. J Neurovirol. 6:284-295]. However, possible relationships and contribution of these genes to the development and course of a BHV5 induced viral encephalitis in ruminants are as yet unknown. In Latin America, BHV5 infections are even seen as the second cause of bovine viral encephalitis, after encephalitis caused by rabies virus. The transmission of BHV5 has not been associated with vampire bats. Like many other heipesvirus infections the virus is transmitted usually trough direct or indirect contact. The major reservoir of the virus identified thus far is cattle, although a role for sheep can not be excluded, since this virus can infect that species experimentally.

As said, another cause of viral encephalitis in ruminants are rabies viruses. Rabies viruses (RV) are members of the Lyssavirus genus of the family of Rhabdoviridae, order Mononegavirales. At present seven genotypes are distinguished among the Lyssaviruses [Bourhy et al. (1993) Molecular diversity of the Lyssavirus genus. Virology. 194:70-81; Gould et al. (1998) Characterisation of a novel lyssavirus isolated from Pteropid bats in Australia. Virus Res. 54:165-187], but also among these genotypes different groups are discerned. Each group associated with different host species. E.g. the "classical" rabies viruses are classified in genotype I along with the rabies viruses belonging to the Chiropteran Lyssaviruses group, which are found in American bats. These bats are either insectivores, frugivores or hematophagous (= vampire bats). The Chiropteran Lyssaviruses of genotype I found in vampire bats cause fatal encephalitis in ruminants like horses and cattle, especially in young calves [Favoretto et al. (2002) Antigenic typing of Brazilian rabies virus samples isolated from animals and humans, 1989-2000. Rev Inst Med Trop Sao Paulo. 44:91-95]. Rabies virus genomes are negative stranded unsegmented RNA molecules of about 12 kb. They code for at least five identified proteins: nucleoprotein 'N', phosphoprotein 'P' (also named 'M' or 'NS' protein), matrix protein 'M' (also named 'M'), glycoprotein G and large protein 'L' [Tordo et al. (1988) Completion of the rabies virus genome sequence determination: Highly conserved domains among the L (polymerase) proteins of unsegmented negative-strand RNA viruses. Virology 165:565-576]. Although the number of rabies cases in dogs, cats and humans in Brazil has decreased in the last decennium, the number of rabies cases in livestock like cattle, transmitted by the vampire bat Desmodus rotundus, has increased during that period [Favoretto et al. (2002) Antigenic typing of Brazilian rabies virus samples isolated from animals and humans, 1989-2000. Rev Inst Med Trop Sao Paulo. 44:91-95]. It was estimated that only in Brazil around 30.000 bovines are lost each year due to a vampire bat transmitted rabies. The number of rabies infected cattle each year in the whole of Latin America must be a multitude of that. Rabies in livestock is named 'paralytic rabies' because paralytic symptoms are the most characteristic signs of the infection. Cattle are infected by vampire bat bites during feeding followed by infection of their central nervous system and eventually rabies infected cattle die of fatal encephalitis. Besides the economic losses rabies infected cattle may be a source of infection for humans.

The invention provides a mutant of bovine herpesvirus 5 (BHV5) provided with a functional deletion in each of at least two genes related to the clinical manifestation of neurovirulence upon infection of a host with BHV5. Such a mutant can be a naturally occurring or so-called spontaneous mutant, which is isolated according to a protocol as given herein, or can be a mutant obtained by recombinant means, as described herein. It has been found that mutating only one gene related to neurovirulence does not result in sufficiently reduced virulence such that a modified life BHV5 vaccine is obtained, at least two genes related to neurovirulence need be functionally deleted. It is preferred that said at least two genes are selected from the group of genes identifiable as the glycoprotein I (gI) gene, the glycoprotein E (gE) gene, the glycoprotein C (gC) gene and the US9 gene, most preferred is that said at least two genes are selected from the group of genes identifiable as the glycoprotein I (gI) gene, the glycoprotein E (gE) gene, and the US9 gene. An additional advantage of deleting two genes is that at least one of the two stands a likely chance to be used as a marker gene, within the context of vaccination against BHV5, it is most preferred that said marker gene function is performed by the glycoprotein E (gE) gene, that thus preferably should be deleted in such a way that no antigenic fragments remain. Such a deletion mutant can be obtained by harvesting virus enriched for gE or gI deletions at a preferred time after infection of a susceptible cell culture with a parent BHV5 strain, as described herein below. The invention allows for construction of a mutant provided with a functional deletion in each of at least three genes related to the clinical manifestation of neurovirulence upon infection of a host with BHV5. It is preferred that said at least one gene comprises the glycoprotein I (gI) gene, the glycoprotein E (gE) gene or the US9 gene, but it for example suffices to fully delete the gE gene and provide for (C-terminal) functional deletions in the gI gene and (N-terminal) functional deletions in the US9 gene. Although it is preferred that said functional deletion provides for the complete lack of expression of a polypeptide encoded by at least one or more of the genes related to the clinical manifestation of neurovirulence upon infection of a host with BHV5 during replication of said mutant in a host, for a functional deletion it is sufficient to provide for a deletion that hampers expression such, or that provides for such a truncated or other ways changed protein (i.e. deletion or insertion of one, two, four, five or more [but not a multitude of three] nucleotides) by frame shift and thus translation of an inappropriate reading frame), that the resulting protein cannot longer perform like the original protein in its interaction with the host. As said, the invention also provides a mutant that is useful as a marker vaccine, in that said preferred complete lack of expression of said polypeptide during replication of said mutant in a host allows for testing of said host and determining whether said host has been infected with a second BHV5 capable of expressing said polypeptide. This is particularly useful when one wants to discriminate animals infected with wild-type BHV5 from animals vaccinated with a mutant vaccine as provided herein. Such testing preferably comprises determining the presence or absence of an antibody specifically directed against said polypeptide in said host. It is preferred that said marker polypeptide comprises gE or gI or both.

In a further preferred embodiment, the invention provides a BHV5 mutant useful as a vector virus. It is herein provided that such a mutant is additionally provided with a nucleic acid encoding an immunogenic (poly)peptide derived from a pathogen other than BHV5. Such a pathogen may be a micro-organism such as a bacterium or protozoa, but preferred is a mutant wherein said pathogen is a virus. In the detailed description herein, an example is provided with the rabies virus. At present two types of rabies vaccines are used to protect cattle against rabies: the attenuated modified live vaccine

Evelyn-Rokitnicki-Albelset = ERA (ERAvac) and the inactivated-adjuvanted PV (Pasteur Virus) vaccine (IPVvac). Both vaccines are based on "classical" or "dog" rabies viruses. If only antibody responses are measured, the inactivated PV vaccine causes high anti-rabies titres in calves after booster and the ERA vaccine doesn't. However, after the use of both the ERA modified live vaccine and the inactivated PV vaccine the detectable anti-rabies antibody titres decline quickly. [Oliveira et al. (2000) Immune response in cattle vaccinated against rabies. Mem Inst. Oswaldo Cruz 95: 83-88]. Although such vaccines were shown to induce antibodies in cattle, the efficacy of such vaccines is, to say the least, doubtful, considering that the value of antibody response data to estimate the efficacy of these vaccines is doubtful, since cell-mediated immunity parameters that correlate with protection against challenge are not evaluated [Zanetti et al. (1998) Failure of protection induced by a Brazilian vaccine against Brazilian wild rabies viruses. Arch.

Virol. 143:1745-1756].

A useful polypeptide gene to insert in a vector mutant as provided by the invention is at least an immunogenic part of protein G of rabies virus. It is of course best to incorporate the full protein G in a mutant as provided herein, however, restricting it to only an immunogenic part is feasible, as long as the resulting part has the desired immunogenicity which can be tested in immunoprecipitation assay or protection experiments. Glycoprotein G is a type I transmembrane protein located in the viral envelope and is implicated in receptor recognition and membrane fusion and needed for axonal transport [Etessami et al. (2000) Spread and pathogenic characteristics of a G-deficient rabies virus recombinant: an in vitro and in vivo study. J Gen Virol. 81:2147-2153]. The coding regions of rabies G protein are about 557 amino acids long, starting with a signal peptide of 20 - 24 amino acids followed by an ectodomain of 450 amino acids, a transmembrane region of 20 amino acids and an endodomain of 50 amino acids. The region between residues 189 to 214 forms the putative binding domain for the nicotinic acetylcholine receptor, which has been identified as an RV receptor [Hanham et al. (1993) Evidence from the anti-idiotypic network that the acetylcholine receptor is a rabies virus receptor. J Virol. 67:530-542]. The G protein of rabies viruses is associated with virulence and especially an arginine or lysine residue on position 333 is important for the virulence function of the G protein [Tuffereau et al. (1989) Arginine or lysine in position 333 of ERA and CVS glycoprotein is necessary for rabies virulence in adult mice. Virology 172:206-212]. The G proteins of rabies viruses are also the main protective antigens [Foley et al. (2000) A recombinant rabies virus expressing vesicular stomatitis virus glycoprotein fails to protect against rabies virus infection. Proc Natl Acad Sci U S A.97:14680-14685]. G proteins induce virus-neutralizing antibodies [Wiktor et al. (1973) Antigenic properties of rabies virus components. J Immunol. 110:269-276], cytotoxic T lymphocytes [Macfarlan et al. (1986) Stimulation of cytotoxic T-lymphocyte responses by rabies virus glycoprotein and identification of an immunodominant domain. Mol Immunol. 23:733-741] and T helper cells [Celis et al. (1988) Recognition of rabies and rabies-related viruses by T cells derived from human vaccine recipients. J Virol. 62:3128-3134].

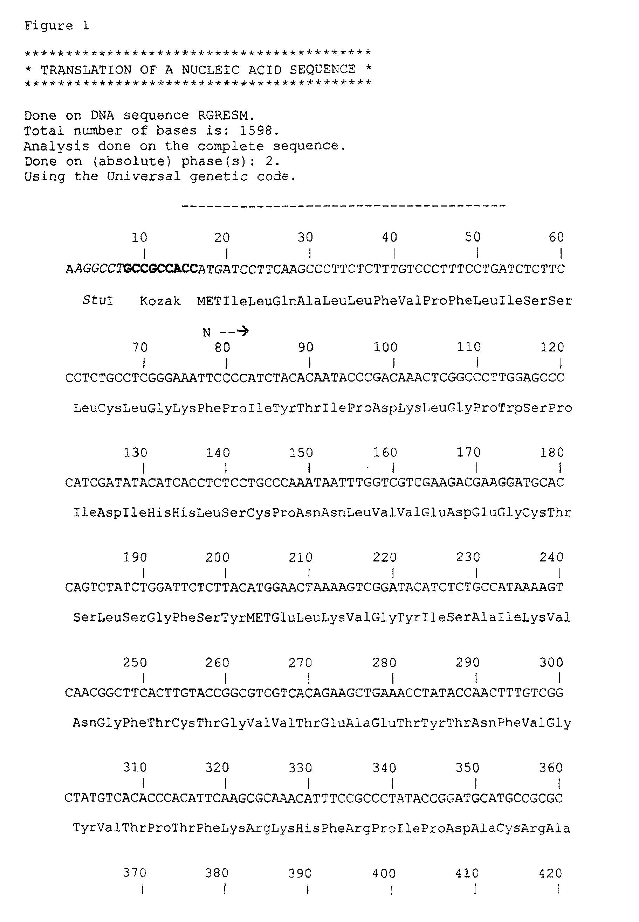

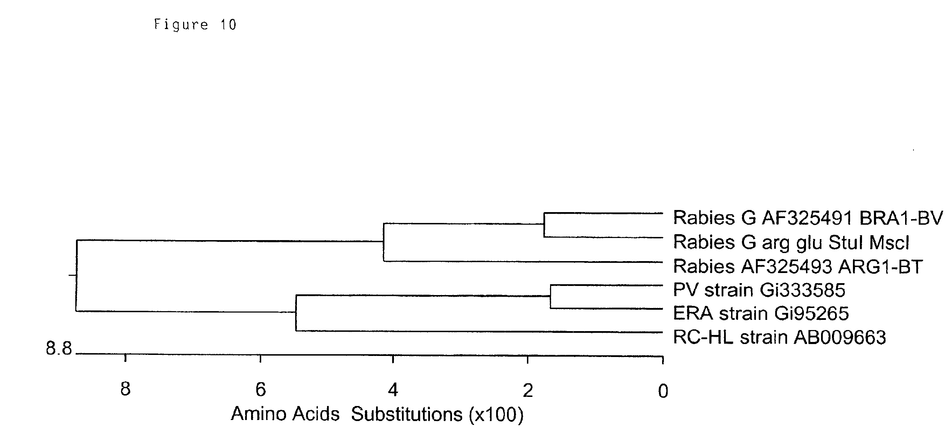

When the presently known 55 Lyssavirus G protein sequences are compared, it can be observed that only 54% of the amino acids are conserved. The G proteins are therefore the primary determinants in the group diversity [Badrane & Tordo (2001), see also figure 10. Host switching in Lyssavirus history from the Chiroptera to the Carnivora orders. J Virol. 75:8096-8104]. However, within each group the degree of amino acid conservation of the G proteins is high. For example within the group of bat rabies viruses belonging to the Chiropteran group of Lyssaviruses of genotype I, the degree of amino acid conservation of the G protein is superior to 95%. [Compare e.g. the bat G protein sequences of isolates ARG1-BT (accession number AF325493), BRA2-BV (accession number AF325491), MEX2-BT (accession number AF325492) and GUY1-BV (accession number AF325490)]. Favoretto et al.(2002, Rev. Inst. Med. trop. S. Paulo 44:91-95) observed that in 100% of the 34 studied cases of rabies in cattle in Brazil, the rabies isolates belonged to the antigenic variant number 3. This is the rabies variant that is found in the hematophagous bat Desmodus rotundus and belongs to the Chiropteran group of Lyssaviruses of genotype I. A vaccine to protect cattle against rabies induced diseases should therefore preferably be directed against this Chiropteran group of Lyssaviruses of genotype I, of which a prototype sequence is given in figure 1. However, in other areas, where other rabies viruses circulate, another G-protein amino acid sequence may be selected. As to the underlying nucleic acid sequence of the selected G protein, it is preferred that it is relatively devoid of predictable U2-splice signals. Furthermore, the invention provides a mutant wherein said (poly)peptide is encoded by a nucleic acid having essentially no predicted splice signal sequences of the U2 type, such a gene herein also called a synthetic gene because it, although encoding an essentially wild-type or nearly wild-type polypeptide, the gene encoding said peptide is devoid of structures that essentially are found in its wild-type counterpart. In a most preferred embodiment, the invention provides a mutant comprising a so-called synthetic gene encoding the G protein of a bat rabies virus. In the detailed description the G protein of a rabies virus belonging to the Chiropteran lyssaviruses isolated from cattle and belonging to the type found in the bat Desmodus rotundus, it being the most immunologically relevant G protein in Latin America. This concerns a bovine herpesvirus 5 (BHV5) mutant with a triple deletion of the glycoprotein I (gI) gene, the glycoprotein E (gE) gene and the US9 gene, based on a South American BHV5 strain. Therewith the invention provides a vector vaccine comprising a BHV5/bat rabies recombinant with the synthetic G protein gene expressed at the site of the gI/gE/US9 deletion. Herewith the invention provides a vaccine comprising a mutant according to the invention. In a preferred embodiment, the invention provides a vaccine against bovine viral encephalitis caused by BHV5, as well as rabies. A said, such a vaccine is preferably based on a synthetic G gene from bat transmitted rabies expressed by a triple deletion mutant of bovine herpesvirus 5. Herewith the invention provides a method of immunizing ruminant hosts against bat rabies and BHV5 induced disease, particularly against viral encephalitis. Furthermore, a method for distinguishing a ruminant infected with a wild-type BHV5 strain or vaccinated against BHV5 from within a population of ruminants vaccinated with a vaccine according to the invention is provided, said method comprises testing a ruminant for the presence or absence of an antibody directed against the glycoprotein I (gI) gene or the glycoprotein E (gE) gene or both.

The invention also provides a method for preparing a vaccine directed against viral encephalitis in ruminants comprising preparing a composition of a mutant as provided herein with a suitable carrier or diluent, and a method for preparing a dual-purpose vaccine directed against viral encephalitis in ruminants and directed against disease caused by another pathogen comprising preparing a composition of a mutant with a suitable carrier or diluent. It is preferred that said vaccine is a modified live vaccine. The invention also provides a test kit provided with the necessary means for performing a method for distinguishing a ruminant infected with a wild-type BHV5 strain or vaccinated against BHV5 from within a population of ruminants vaccinated with a marker vaccine as provided herein in the detailed description said method comprising testing a ruminant for the presence or absence of an antibody directed against the glycoprotein I (gI) gene or the glycoprotein E (gE) gene or both.

Considering that viral encephalitis due to bat transmitted rabies and due to BHV5 is a growing problem among ruminants, especially cattle, mainly in South America, that the presently used cattle rabies vaccines fail to confer sufficient protection against bat transmitted rabies and a vaccine against BHV5 is not available and that the suggested cross-protection of BHV1 is not sufficient for more virulent BHV5 strains, the invention described here provides a vaccine 1) against bat rabies to protect ruminants, more specifically cattle, against bat rabies induced diseases, more specifically against viral encephalitis, 2) against bovine herpesvirus 5 (BHV5) to protect ruminants, more specifically cattle against BHV5 induced diseases, more specifically against viral encephalitis. A region specific embodiment of the vaccine provided here comprises a so-called synthetic gene encoding the G protein of a bat rabies virus belonging to the Chiropteran lyssaviruses isolated from cattle and belonging to the type found in the bat Desmodus rotundus. It is preferred to use as vaccine a BHV5 mutant with a triple deletion of the glycoprotein I (gI) gene, the glycoprotein E (gE) gene and the US9 gene, based on a South American BHV5 strain. This triple deletion is sufficiently safe while still sufficiently immunogenic. It is preferred that the synthetic G protein gene is inserted at the site of the gI/gE/US9 deletion.

The present invention provides a specific vaccine against bat transmitted rabies and against BHV5 for cattle, resulting in a tool to reduce bovine viral encephalitis and decreasing the costs for the farmers.

### LEGENDS TO FIGURES

### Figure 1

The 1598 nucleotides long sequence of the synthetic DNA fragment RGRESM which codes for the G protein found in the "GUY1-BV" rabies isolated in French Guyana in 1985 from a bovine host infected by a rabies variant that has Desmodus rotundus as a host. The synthetic DNA fragment RGESM encoding this bat rabies G protein is double stranded, but in this figure only the protein coding strand is given. This nucleotide sequence is translated according to the universal code and the encoded amino acids are indicated in three letter codes below the nucleotide sequence. This amino acid sequence is identical (with one exception) to the translated sequence retrieved from the National Center for Biotechnology Information (NCBI) nucleotide database, accession number AF325490. This sequence was submitted (01-DEC-2000) by the Department of Virology, Laboratoire des Lyssavirus, Institut Pasteur, 25, rue du Dr. Roux, Paris Cedex 15 75724, France. And it was also mentioned in the publication of H. Badrane and N. Tordo (2001) Host switching in Lyssavirus history from the Chiroptera to the Carnivora orders. J. Virol. 75 (17), 8096-8104.

The one exception is amino acid with residue number 352. This amino acid, which was in the original amino acid sequence an arginine (Arg) residue, has been substituted by a glutamine (Glu) residue. This substituted amino acid residue has been indicated in bold and by a star (*). The amino acid sequences being almost identical to the GUY1-BV sequence mentioned above, the nucleotide sequence is however changed in many places (about 10%). These nucleotide changes were introduced to remove the common or U2-type of splicing signals (Sharpe & Burge 1997 Classification of introns: U2-type or U12 type, Cell 91, 875-879.).

The start codon (ATG) of this bat rabies G protein encoding region is pointed out by an N with an arrow, indicating the N-terminus of this bat rabies G protein. The last codon (CTC) is pointed out by an arrow and a C, indicating the C-terminus of this G protein. That last codon is followed by the stop codon (TGA) and this codon is indicated by the word 'stop'. Upstream of the start codon (ATG) a Kozak consensus sequence is inserted indicated in bold. To both sides of the DNA fragment the recognition sequence of restriction enzyme StuI is added that allow the use of restriction enzyme StuI to take the bat rabies G encoding sequence together with the Kozak consensus sequence and the stop codon out of a cloning vector (e.g. pGEM-T-easy).

### Figure 2

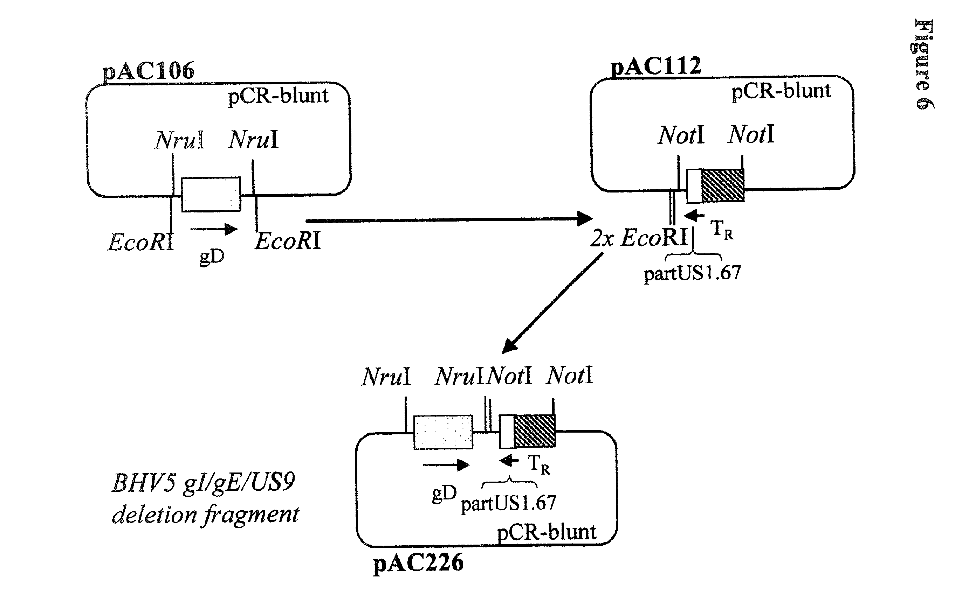

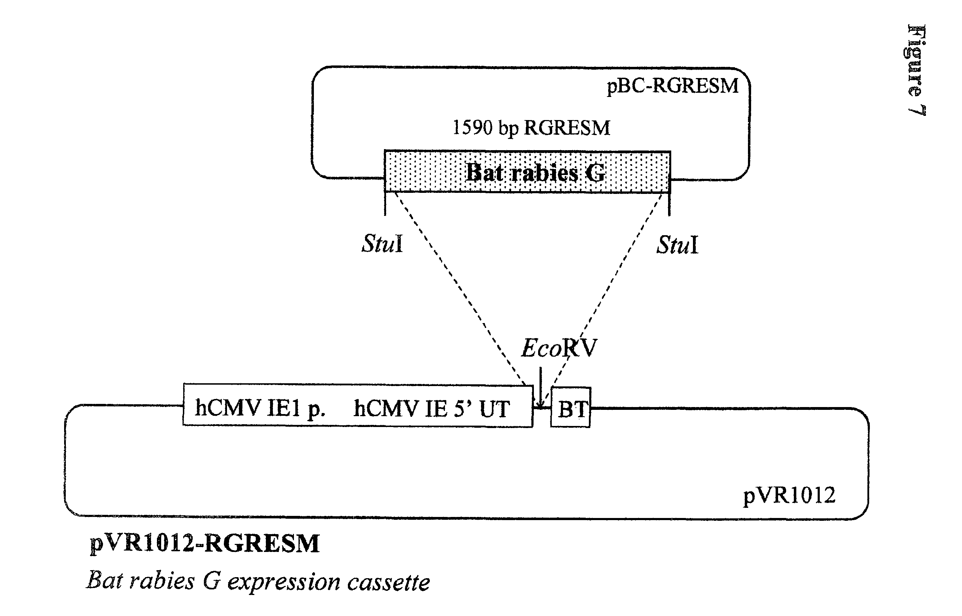

Structure of plasmid pBC-RGRESM. The 1598 base pairs (bp) long DNA fragment RGRESM was cloned in prokaryotic plasmid pGEM-T easy. By digesting plasmid pBC-RGRESM with restriction enzyme StuI a 1590 nucleotides long fragment was liberated that encodes the bat rabies G protein of which the amino acid sequence is given in Figure 1. This 1590 bp fragment was inserted into the expression cassette of plasmid pVR1012. See Figure 7.

### Figure 3

Diagram of the structure of the BHV5 genome and the genomic organisation of the genes of the recombination region. Wild type BHV5 is approximately 140 kilo base pairs (kb) long and has an Unique Long (U_{L}) and an Unique Short (U_{S}) region. The Unique Short is bordered by an inverted repeat indicated by hatched boxes (I_{R} = internal repeat T_{R} = terminal repeat). A physical map of the Unique Short region of wild type (wt) strain EVI-88 (BHV5 strain isolated in Brazil in 1995) has been shown in more detail under wt (EVI-88). The indicated region is a 16.4 kb long BamHI fragment on which the genes are indicated that code for glycoprotein D (gD), glycoprotein I (gI) and glycoprotein E (gE) and the US9 gene. The protein encoding region of these genes has been shown by boxes indicated with their respective names. The arrows beneath these boxes indicate the direction of transcription of these genes. Downstream of the US9 gene a partial duplication has been found of the US1.67 gene (partUS1.67). Next to the restriction enzyme recognition sites for BamHI, the recognition sites for EcoNI are also indicated.

### Figure 4

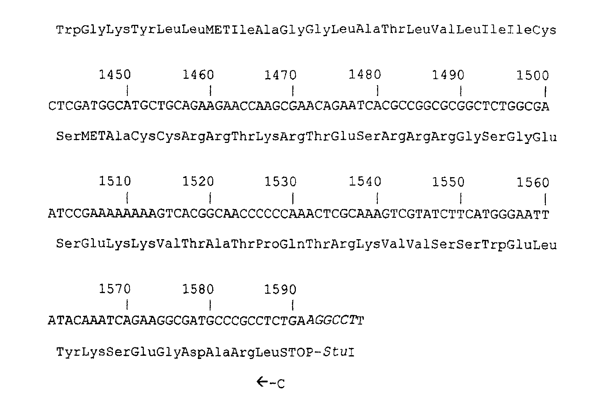

Diagram of the cloning and subcloning of the US region of BHV5 in order to construct a recombination fragment that deletes BHV5 genes gI/gE and US9 and replaces these genes by an expression cassette for bar rabies G protein. The top indicates plasmid pAC41 which harbours the 16.4 kb BamHI fragment of BHV5 strain EVI-88 cloned into the BamHI site of pBR322. Besides the BamHI sites, the EcoNI sites are indicated together with the genes coding for gD, gI, gE and US9 (see also legends of Figure 3). From the pAC41 clone the 5.8 kb EcoNI fragment encoding the gD, gI and gE genes has been subcloned into the blunt site of pCR-Blunt resulting in plasmid pAC104 and the 3.6 kb EcoNI-BamHI/EcoN1 fragment has been subcloned into the blunt site of pCR-Blunt resulting in plasmid pAC102. partUS1.67 is a partial sequence of the US1.67 gene. T_{R}= terminal repeat.

### Figure 5

Diagram of the subcloning of the recombination fragments upstream and downstream of the BHV5 gI/gE/US9 deletion. From pAC104 a 1.8 kb NruI fragment, encoding the C-terminal part of the gG glycoprotein (partgG) and the complete gD glycoprotein (gD) was cloned into the blunt site of pCR-Blunt, resulting in plasmid pAC106. From pAC 102 a 1.6 kb NotI fragment was subcloned into the NotI site of a circular form of pCR-Blunt, resulting in plasmid pAC112. The NruI and NotI sites are indicated. The BHV5 genes are indicated as explained in the legends of Figure 3. The two BHV5 fragments were cloned into their respective vectors in the orientations as indicated in the diagrams. T_{R} = (part of) terminal repeat.

### Figure 6

Diagram of the construction of the BHV5 gI/gE/US9 deletion fragment. The 1.8 kb Nrul fragment has been liberated from plasmid pAC106 using restriction enzyme EcoRI that cuts in the pCR-Blunt vector close to the insertion site (less than 10 bp on each side). The 1.8 NruI fragment together with the EcoRI ends of the pCR-Blunt vector was cloned into the EcoRI site of pAC112 that was formed after digesting pAC112 with EcoRI (This removed the blunt-end cloning region between the EcoRI sites, but left the neighbouring 1.6 kb NotI fragment intact).

A clone with the NruI fragment in the same orientation with respect to the NotI fragment as in the BHV5 genome and as depicted in the diagram was named pAC226. The constructed 3.4 kb NruI/NotI fragment in pAC226 can be used to recombine with wild type BHV5 genome to propagate a BHV5 gI/gE/US9 deletion.

### Figure 7

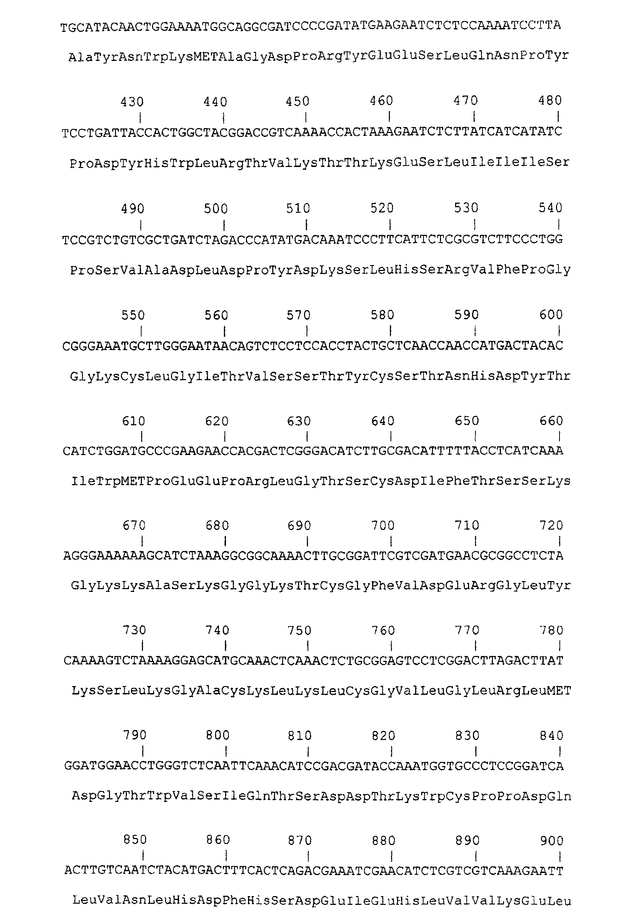

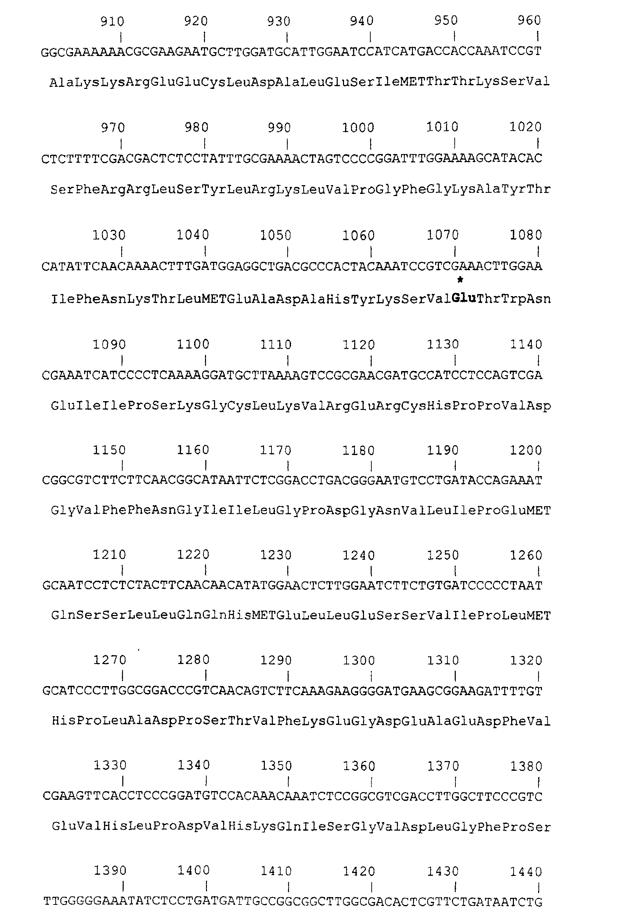

Diagram of the construction of the hCMViel expression cassette to express bat rabies G protein. The 1590 bp DNA fragment that is cut out of plasmid pBC-RGRESM (See Figure 2) by restriction enzyme StuI has been cloned into the EcoRV site of eukaryotic expression vector pVR1012 in such an orientation that the N-terminus (See Figure 1) is at the side of the promoter and the C-terminus (See Figure 1) is at the side of the BT sequence. The promoter in pVR1012 is the human cytomegalovirus IE1 promoter/enhancer region (hCMV IE1 p.) which is followed by the 5' untranslated leader (hCMVIE 5' UT). Downstream of the insertion site of pVR1012 is the bovine growth hormone terminator sequence (BT).

### Figure 8

Diagram of the cloning of the hCMVielexpression cassette containing the bat rabies G protein into the 3.4 kb BHV5 deletion/recombination fragment. The hCMViel expression cassette containing the 1590 bp DNA fragment encoding the bat rabies G protein has been cut from plasmid pVR1012-RGRESM using restriction enzymes MscI and Acc65I. The MscI-Acc65I fragment is 3.6 kb long (In this and the following diagram the scale of this 3.6 kb 5 times the scale of the 3.4 NruI-NotI BHV5 deletion/recombination fragment.) The Acc65I site has been made blunt and the 3.6 kb MscI-Acc65I fragment is cloned into the EcoRV site of pAC226. The bat rabies expression cassette has in the resulting plasmid the same transcription orientation as the gD gene and the original gI/gE/US9 genes, as indicated by the arrows beneath the gD gene and bat rabies G gene.

### Figure 9

Diagram of the resulting BHV5-gI·/gE·/US9·bat rabies G expression vector named BHV5-hCMV-RGRESM.

The top indicates the overall structure of the approximately 140 kb BHV5 genome, with its Unique Long (U_{L}) and it Unique Short (U_{S}) regions. The Us region is bordered by an inverted repeat indicated by hatched boxes (I_{R} = internal repeat T_{R}= terminal repeat).

In the middle the diagram show the BHV5 Us region after the 4 kb deletion of the gI/gE and US9 gene, resulting in a BamHI fragment of 12.4 kb. Between the gD gene and the partial US1.67 gene at an EcoRV site origination from pAC226, the bat rabies G hCMV expression is inserted.

The bottom indicates the 3.6 kb MscI-Acc65I fragment harbouring the bat rabies G expression cassette as it is inserted in the BHV5 genome ate the site of the gI/gE/US9 deletion.

### Figure 10

Phylogenetic tree based on an alignment by the ClustalV method of the amino acid sequences of three bat rabies G sequences and three G sequences of 'classical' rabies vaccines strains. The bat rabies sequences are from Argentinean isolate ARG1-BT (NCBI accession number AF325493 ), Brazilian isolate BRA1-BV (NBCI accession number AF325491) and the bat rabies G sequence, as given herein. The classical rabies vaccine sequences are from the ERA strain, the PV strain and Japanese rabies vaccine strain RC-HL (with respective NCBI accession numbers Gi95265, Gi333585 and AB009663). The tree shows that the bat rabies G sequences form a cluster separated from the G sequences from the 'classical' rabies vaccines.

### DETAILED DESCRIPTION

### Design of a synthetic gene encoding a suitable bat rabies G protein

To chose an amino acid sequence of a bat rabies G protein suitable to be used in a vaccine to protect against bat rabies transmitted by the haematophagus bat Desmodus rotundus, a number of G protein sequences of recent bat rabies isolates were compared. These included the Argentinean isolate ARG1-BT (accession number AF325493) isolated from a bat in Argentina in 1991, Brazilian isolate BRA2-BV (accession number AF325491) isolated from cattle in Brazil in 1986, Mexican isolate MEX2-BT (accession number AF325492) isolated from a bat in Mexico in 1987, and GUY1-BV (accession number AF325490) isolated from cattle in French Guyana in 1985. All four G protein sequences were aligned with each other together with the French isolate FRA1-FX (accession number AF325461) isolated from a fox in 1991, using the ClustalW program of sequence analysis program DNASTAR. This analysis showed that all four bat G protein sequences clustered together and excluded the French fox isolate. See also the results of Badrane and Tordo (2001) Host switching in Lyssavirus history from the Chiroptera to the Carnivora orders. J. Virol. 8096-8104. The BRA2-BV and the GUY1-BV could both be used as a starting sequence for the synthetic gene, being both isolated from bovine and having both the bat Desmodus rotundus as a variant host.

However, the BRA2-BV sequence shows some aberrant substitutions between amino acids 470 and 500. Therefore, the GUY1-BV G protein sequence was taken as a starting point to design a synthetic bat rabies G sequence. As the mRNA of the rabies virus is normally not present in the nucleus of the infected cells, the coding region of the rabies G of GUY1-BV had to be screened for the presence of common or U2-type of splicing signals (Sharpe & Burge 1997 Classification of introns: U2-type or U12 type, Cell 91, 875-879.). Using the sequence analysis program PC-gene for the detection of splicing signals, 12 splice donor signals or exon/intron borders and 5 splice acceptor signals or intron/exon borders were discovered that scored above the standard cut-off values of the program. These splicing signals, when expressed by BHV5 in the nucleus, could be recognised and processed by U2 type spliceosomes. Therefore, the sequence was adapted in such a way that all significant splicing signals, but also a large number of spicing signals that scored under the cut-off value, were removed while the coding potential remained unaffected [Kuhnle et al. (1998) The class II membrane glycoprotein G of bovine respiratory syncytial virus, expressed from a synthetic open reading frame, is incorporated into virions of recombinant bovine herpesvirus 1. J Virol. 72:3804-11].

The adapted nucleotide sequence has been designed in such a way that the resulting DNA sequence codes for the same amino acids as the originally GUY1-BV G protein sequence with the exception of amino acid residue number 352. This amino acid, which was in the original amino acid sequence an arginine (Arg) residue, has been substituted by a glutamine (Glu) residue. This substitution of residue 352, or residue 333 of the mature protein after removal of the signal peptide, reduces the virulence of the G protein [Tuffereau et al. (1989) Arginine or lysine in position 333 of ERA and CVS glycoprotein is necessary for rabies virulence in adult mice. Virology 172:206-12; Benejean et al. (1998) Avirulent anti-rabies vaccine. US patent US5853735 dated 1998-12-29].

To stimulate efficient translation of the encoded bat G protein, a consensus Kozak sequence (GCCGCCACC) was added upstream of the coding sequence immediately before the ATG start codon. [M. Kozak (1986) Point mutations define a sequence flanking the AUG initiator codon that modulates translation by eukaryotic ribosomes. Cell 44:283-92]. At the end of the protein coding region, a stop codon was inserted behind the carboxy-terminal end of the G protein. To allow the removal of the designed sequence in the form of a blunt ended fragment from a cloning plasmid, the sequence recognised by the StuI restriction enzyme, was added to both sides of the designed sequence bringing the total length to 1598 nucleotides. See Figure 1. Using standard DNA synthesising methods, a double stranded DNA fragment of 1598 nucleotides was synthezised of the designed sequence, cloned into prokaryotic plasmid pGEM-T-easy and propagated in Escherichia coli bacteria. See figure 2.

Cloning of the Unique Short region of bovine herpesvirus 5 (BHV5) strain EVI-88.

Bovine herpesvirus 5 is highly neurovirulent for cattle with fatal consequences. To develop a BHV5 vector that has sufficient efficacy as a vaccine, the South American BHV5 isolate EVI-88, isolated in Rio Grande do Sul in Brazil in 1995, was taken as a parent strain. This strain was preferred above isolates from other continents like Australia, because of possible antigenic differences. When studied by restriction enzyme analysis, BHV5 strain EVI-88 shows differences with respect to the Australian N569 strain. Moreover, BHV5 strain is a representative of the majority of the BHV5 isolates identified in South America thus far. To construct a BHV5 vector that is sufficiently attenuated, genes that were identified in playing a role in the neurovirulence of BHV5 were deleted from the genome of BHV5 strain EVI-88. Thus, the gene coding for glycoprotein E (gE) was identified to play a role in neuroinvasiveness and neurovirulence in the olfactory pathway [Chowdhury et al. (2000) Bovine herpesvirus 5 glycoprotein E is important for neuroinvasiveness and neurovirulence in the olfactory pathway of the rabbit. J Virol. 74:2094-2106] and was therefore deleted. The US9 gene of BHV5 has also been implicated in neurovirulence because of its role the in transport of viral glycoproteins along the axons [Chowdhury et al. (2002) Bovine herpesvirus 5 (BHV-5) Us9 is essential for BHV-5 neuropathogenesis. J Virol. 76:3839-3851] and was also deleted.

To further attenuate BHV5 strain EVI-88 the gene encoding glycoprotein I (gI) was also deleted. Glycoproteins gI an gE form a functional complex, and therefore may affect the same role in the cell-to-cell spread mechanisms of BHV5. However, gI may have additional functions because a gI/gE double deletion mutant of BHV1 infection behaved more attenuated as the single deletion mutant of both genes [Kaashoek et al. (1998) Virulence, immunogenicity and reactivation of bovine herpesvirus 1 mutants with a deletion in the gC, gG, gI, gE, or in both the gI and gE gene. Vaccine. 16:802-809]. The site of the gI/gE/US9 deletion is also used to insert the bat rabies G protein expresion cassette.

It was expected that all three genes (gI, gE and US9) were located next to each other at one side of the Unique Short region of the BHV5 genome. To create this triple deletion mutant of BHV5 strain EVI-88, the 16.4 kilo base pairs (kb) BamHI fragment, which covers the complete Unique Short region of BHV5 [Engels et al., (1986) The genome of bovine herpesvirus 1 (BHV-1) strains exhibiting a neuropathogenic potential compared to known BHV-1 strains by restriction site mapping and cross-hybridization. Virus Res. 6:57-73; Bulach & Studdert (1990) Comparative genome mapping of bovine encephalitis herpesvirus, bovine herpesvirus 1, and buffalo herpesvirus. Arch. Virol. 113:17-34] was cloned into pBR322. See figure 3.

### Localisation of the putative positions of the gG, gD, gI, gE and US9 genes on the 16.4 BamHI fragment of EVI-88

To construct a recombination fragment that allows both the deletion of the gI/gE/US9 genes and the simultaneous substitution of the bat rabies G protein expression cassette, the putative positions of the gI, gE and US9 genes of the BHV5 strain EVI-88 were identified within the 16.4 kb BamHI fragment. Based on the published data about the localisation of the BHV5 gG (also named US4) [Engelhardt & Keil (1996) Identification and characterization of the bovine herpesvirus 5 US4 gene and gene products. Virology 225:126-135. NCBI accession number X99755] and on BHV5 gD [Abdelmagid et al. (1995) Fine mapping of bovine herpesvirus-1 (BHV-1) glycoprotein D (gD) neutralizing epitopes by type-specific monoclonal antibodies and sequence comparison with BHV-5 gD. Virology 206: 242-253, NCBI accession number U14656], it was inferred that the EcoNI site that Engelhardt and Keil (1996) found in the middle of the gG gene of BHV5 strain N569 was conserved in the EVI-88 strain and that the gD gene was located on the left end of the 5.8 kilo base pairs EcoNI fragment in the centre of the 16.4 kilo base pairs BamHI fragment of the genome of the EVI-88 strain. See Figure 3. To identify the positions of the gE gene and the US9 gene within the 16.4 kb BamHI fragment of the EVI-88 strain, the published sequence data of Chowdhury et al. (2000 and 2002) were used [Chowdhury et al. (2000) Bovine herpesvirus 5 glycoprotein E is important for neuroinvasiveness and neurovirulence in the olfactory pathway of the rabbit J. Virol. 74: 2094-2106, NCBI accession number AF208294 and Chowdhury et al. (2002) Bovine herpesvirus 5 (BHV-5) Us9 is essential for BHV-5 neuropathogenesis J. Virol. 76 (8), 3839-3851, NCBI accession number AY064172]. See figure 3.

### Subcloning of the gD, gI, gE, US9 region BHV5 strain EVI-88

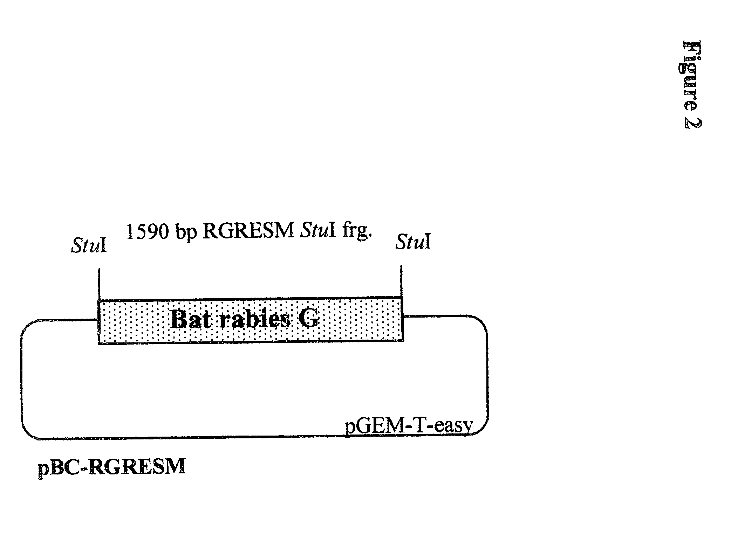

To further characterize the physical map of the Unique Short region of BHV5 strain EVI-88 and to have suitable subclones to construct a deletion fragment for the gI/gE/US9 region, two subclones were constructed from the 16.4 kb BamHI clone pAC41. First pAC41 was digested with the EcoNI, liberating a 3.6 kb EcoNI-BamHI/EcoNI fragment because of an EcoNI site located in the pBR322 vector next to the BamHI site in which the 16.4 kb fragment was cloned. See Figure 4. This EcoNI fragment was made blunt using Klenow polymerase and was ligated into the pCR-Blunt vector using standard methods. The resulting clone was named pAC 102. See Figure 4. The same EcoNI digest of pAC41 also liberated a 5.8 kb EcoNI fragment originating from the middle of the 16.4 kb BamHI fragment. This 5.8 kb EcoNI fragment was also made blunt and cloned into pCR-Blunt using standard methods. The resulting clone was named pAC104. See Figure 4. Both clones were used to perform further restriction enzyme analyses to locate the putative position of the genes in this region of BHVS strain EVI-88.

### Construction of the BHV5 deletion/recombination fragment for the gI/gE/US9 locus of BHV5.

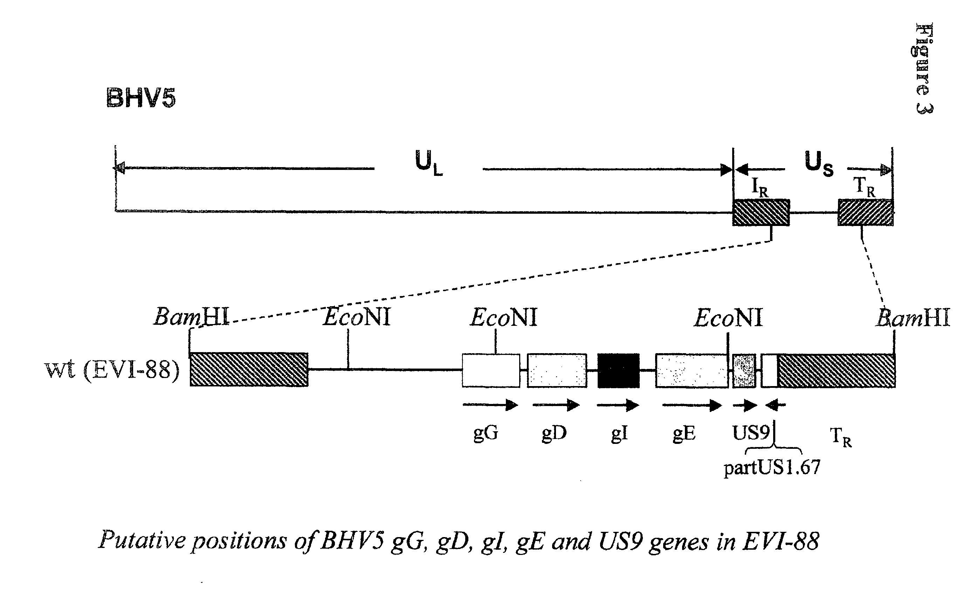

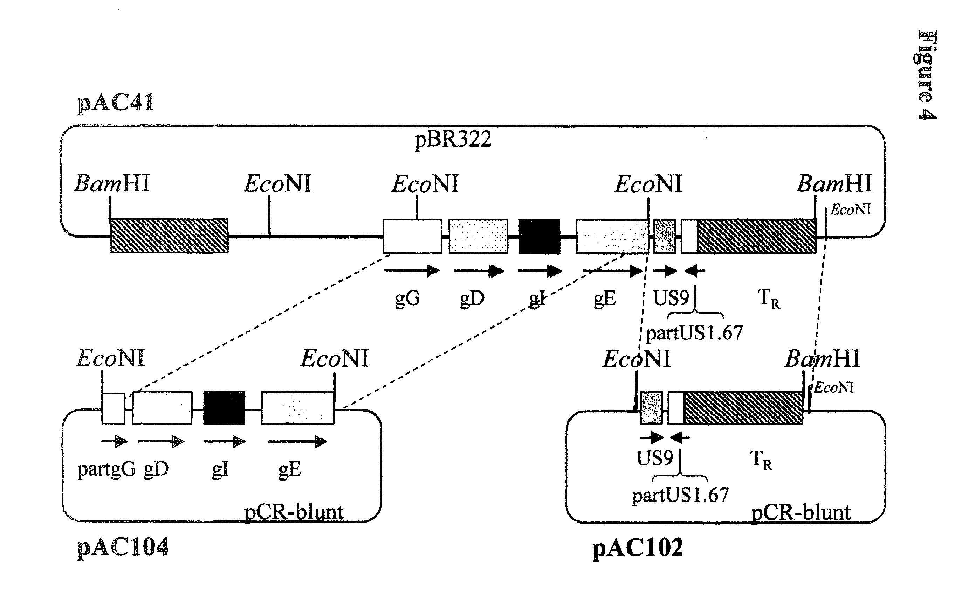

To construct a recombination fragment that can be used to delete the gI/gE/US9 region form the genome of BHV5 strain EVI-88, both an upstream fragment and a downstream fragment had to be cloned. Both fragments should be at least 1 kb long to allow recombination with the wild type genome and both fragments should border at the site of the intended deletion. The upstream fragment that was chosen was the 1.8 kb NruI fragment that starts 149 nt upstream of the end of the gG coding region, covers the complete gD coding region and ends at the start of the gI coding region. See Figure 5.

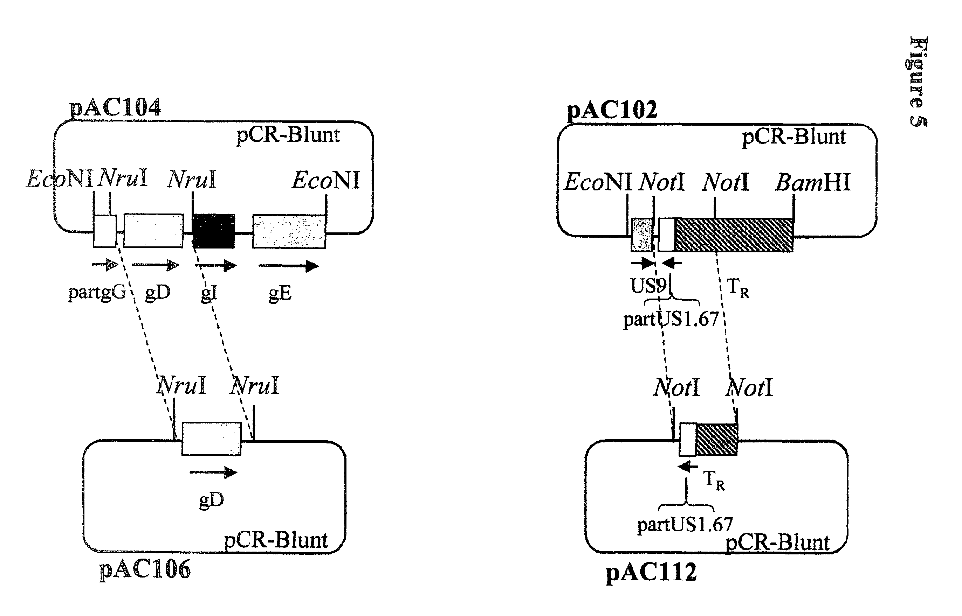

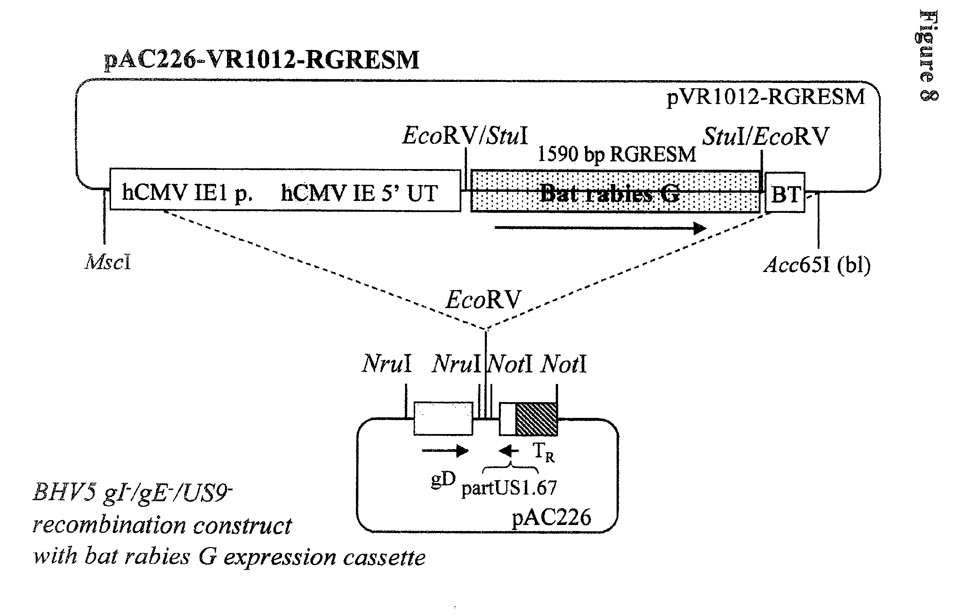

The downstream fragment that was chosen was 1.6 kb NotI fragment that starts at the end of the US9 coding region, covers the partial US 1.67 gene and ends in the terminal repeat region. Both fragments were cloned into pCR-Blunt using standard methods. The NruI site, which is blunt, was cloned into the blunt site of pCR-Blunt, resulting in clone pAC106 and the 1.6 NotI fragment was cloned into the NotI site of a cloned pCR-Blunt plasmid digested with NotI. The resulting clone was named pAC112. To construct the deletion/recombination fragment to delete the gI, gE and US9 genes form BHV5 strain EVI-88, both the upstream and the downstream fragments were cloned next to each other in the same orientation as found in the original 16.4 BamHI Unique Short fragment. To this end the upstream 1.8 kb NruI fragment was liberated from clone pAC106 using the EcoRI sites that border the insertion site of pCR-Blunt. See Figure 6. This 1.8 kb EcoRI/NruI-NruI/EcoRI fragment was cloned into the EcoRI site of clone pAC 112 next to the NotI site that contains the downstream fragment. The resulting clone is named pAC226 and has both the upstream fragment and the downstream fragment cloned next to each other. See figure 6. The upstream fragment covering the complete gD coding region was only separated by a small part of the multiple cloning site of pCR-Blunt (about 30 nucleotides between the blunt site to the NotI site) from the down stream fragment covering the partial US1.67 gene and part of the terminal repeat region. The short part of this multiple cloning region of pCR-Blunt contained a unique EcoRV site that allowed the insertion of the bat rabies G protein expression cassette. See Figure 8.

### Cloning the bat rabies G gene into eukaryotic expression vector pVR1012.

For the expression of the bat rabies G protein as encoded by the nucleotide sequence given in Figure 1, the 1590 bp StuI fragment of plasmid pBC-RGRESM as depicted in Figure 2, was cloned into the EcoRV site of expression vector pVR1012 in the same orientation as the cis-regulatory sequences present on this eukaryotic expression vector. In the resulting construct, named pVR1012-RGRESM, the bat rabies G protein encoding region is located downstream of the human cytomegalovirus immediate early 1 promoter (hCMViel promoter) and its 5' untranslated leader (5'UT), and upstream of the bovine growth hormone terminator sequence (BT). See Figure 7. The hCMViel promoter is known to be active in many types of eukaryotic cells and also in the background of an alphaherpesvirus infection. The 5'UT region contains an intron bordered by canonical splice donor and splice acceptor signals of the U2 type, stimulating the export of mRNA from the nucleus and possibly competing for undesirable splicing events in the bat rabies G protein encoding region. The BT sequence helps to avoid that the pre-mRNA transcribed from this expression cassette will become too long and contains a signal for processing at a polyadenylation site close behind the coding region. Both regulatory events improve the stability of the mRNA and lead to a higher expression of the encoded protein.

### Cloning the bat rabies expression cassette into BHV5 deletion/recombination plasmid pAC226.

To allow the recombination of the bat rabies G protein expression cassette into the genome of BHV5 strain EVI-88 at the site of the gI, gE and US9 genes, the bat rabies G protein expression cassette was cloned into the deletion/recombination plasmid pAC226, right between the upstream and the downstream fragments. For this, the bat rabies G protein expression cassette was cut out of the pAC226-VR1012-RGRESM clone using restriction enzymes MscI and Acc65I . The resulting 3.6 kb MscI-Acc65I fragment was made blunt and cloned into the EcoRV site located between the upstream and the downstream recombination fragments in pAC226. The bat rabies G protein expression cassette was cloned in the same orientation as the upstream gD gene and the original gI. gE and US9 genes which it replaced. See Figure 8.

### Construction and isolation of the BHV5 gI/gE/ US9 deletion mutant expression the coding region for bat rabies G protein named BHV5-hCMV-RGRESM-ACF7

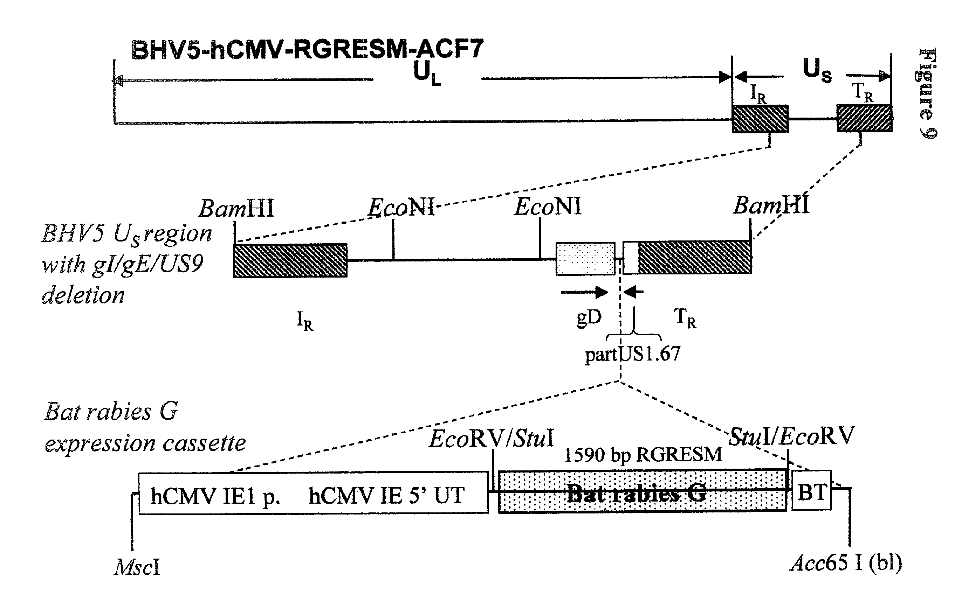

The construction of the BHV5/bat rabies G recombinant (as described in Figure 9) that expresses the bat rabies G protein from which the amino acid sequence has been indicated in Figure 1 was performed as follows. First linearized plasmid pAC266-VR1012-RGRESM as shown in Figure 8 was co-transfected together with purified genomic DNA of parent BHV5 strain EVI-88 into embryonic bovine trachea cells (EBTr cells) using the calcium phosphate-mediated transfection method of Graham & van der Eb (1973, A new technique for the assay of infectivity of human adenovirus 5 DNA. Virology. 52:456-467) and 48 hours after co-transfection cells were freeze/thawed to liberate putative recombinant viruses. To isolate such recombinant viruses, the cell debris was pelleted and the supernatant was used to infect new monolayers of EBTr cells on a 96 wells plate and to identify bat rabies G positive wells using an immunostaining procedure with anti-bat rabies G protein antisera. The virus in the medium of wells positive for anti-bat rabies G protein antisera was diluted to infect bovine cells on another 96 wells microplate with monolayers of EBTr cells. Virus from bat-rabies G protein positive single BHV5 plaques was three times plaque purified to obtain a purified BHV5/bat rabies G protein recombinant. One of the recombinants obtained this way, was named BHV5-hCMV-RGRESM ACF7. This virus is a triple deletion mutant with respect to the genes coding for glycoproteins gI and gE and for the US9 protein. And it carries the hCMV expression cassette containing a synthetic gene encoding a bat rabies G protein at the site of this triple deletion. See Figure 9.

### Isolation of a natural gI/gE/US9, gE/US9 or US9 deletion mutant of BHV5

To isolate a spontaneous or natural gI/gE/US9 deletion mutant of BHV5 the following protocol applies. The protocol is based on the insight that such deletion mutants are preferably released earlier during replication from an infected cell than the parent virus. If required, firstly a point in time may be determined at which a constructed gI/gE/US9 deletion mutant is secreted better from infected EBTr cells than parent strain EVI-88 or another strain of BVH5. However, this is not necessary, it suffices to harvest virus during a clearly restricted period in which this preferred secretion takes place. To better accommodate this, the infection time is preferably limited. Therefore, EBTr or other susceptible bovine cells are infected with wild type BHV5 strain (e.g. EVI-88) at an MOI of 10 and 1 hour after infection the infected cells are treated with sodium nitrate pH3.0, to inactivate the non-penetrated virus. At the optimal time post infection, which depending on the culture conditions is between 9-11 hours after the infection, the tissue culture medium of the infected cells is collected and used to infect another monolayer of susceptible cells. This is done to expand the virus population that is enriched for spontaneous gI/gE/US9 deletion mutants of BHV5. This virus population is used for another round of enrichment. Optimally, in total 5- 8 rounds of enrichment are accomplished and from the ultimately obtained population 400-600 plaques are isolated and tested for the expression of gI using anti-gI peptide sera to identify a gI negative virus. The gI negative viruses identified are further tested for the expression of gE with anti-gE-peptide sera and for US9 with an anti-US9-peptide sera. Resulting gI/gE/US9 negative BHV5 viruses are three times plaque purified and the size of the deletion is studied restriction enzyme analysis. A similar protocol applies for isolating gE/US9 or US9 deletion mutants of BHV5.

## Claims

1. A mutant of bovine herpesvirus 5 (BHV5) provided with a functional deletion in each of at least two genes related to the clinical manifestation of neurovirulence upon infection of a host with BHV5.

2. A mutant according to claim 1 wherein said at least two genes are selected from the group of genes identifiable as the glycoprotein I (gI) gene, the glycoprotein E (gE) gene, the glycoprotein C (gC) gene and the US9 gene.

3. A mutant according to claim 2 wherein said at least two genes are selected from the group of genes identifiable as the glycoprotein I (gI) gene, the glycoprotein E (gE) gene, and the US9 gene.

4. A mutant according to claim 2 or 3 provided with a functional deletion in each of at least three genes related to the clinical manifestation of neurovirulence upon infection of a host with BHV5.

5. A mutant according to claim 4 provided with a functional deletion in each of the glycoprotein I (gI) gene, the glycoprotein E (gE) gene and the US9 gene.

6. A mutant according to anyone of claims 1 to 5 wherein said functional deletion provides for the complete lack of expression of a polypeptide encoded by at least one of the genes related to the clinical manifestation of neurovirulence upon infection of a host with BHV5 during replication of said mutant in a host.

7. A mutant according to claim 6 wherein said at least one gene comprises the glycoprotein I (gl) gene, the glycoprotein E (gE) gene or the US9 gene.

8. A mutant according to claim 6 wherein said complete lack of expression of said polypeptide during replication of said mutant in a host allows for testing of said host and determining whether said host has been infected with a second BHV5 capable of expressing said polypeptide.

9. A mutant according to claim 8 wherein said second BHV5 is wild-type.

10. A mutant according to claim 8 or 9 wherein said testing comprises determining the presence or absence of an antibody specifically directed against said polypeptide in said host.

11. A mutant according to anyone of claims 1 to 10 additionally provided with a nucleic acid encoding an immunogenic (poly)peptide derived from a pathogen other than BHV5.

12. A mutant according to claim 11 wherein said pathogen is a virus.

13. A mutant according to claim 12 wherein said virus is rabies virus.

14. A mutant according to claim 13 wherein said (poly)peptide comprises a part of protein G of rabies virus.

15. A mutant according to claim 14 wherein said protein G is derived of a bat rabies virus.

16. A mutant according to anyone of claim 11 to 15 wherein said (poly)peptide is encoded by a nucleic acid having essentially no predicted splice signal sequences of the U2 type.

17. A method for preparing a vaccine directed against viral encephalitis in ruminants comprising preparing a composition of a mutant according to anyone of claims 1 to 10 and a suitable carrier or diluent.

18. A method for preparing a dual-purpose vaccine directed against viral encephalitis in ruminants and directed against disease caused by another pathogen comprising preparing a composition of a mutant according to anyone of claims 11 to 16 and a suitable carrier or diluent.

19. A method according to claim 18 wherein said other pathogen is rabies.

20. A method according to claim 17 to 19 wherein said vaccine is a modified live vaccine.

21. A vaccine comprising a mutant according to anyone of claims 1 to 16.

22. A method for distinguishing a ruminant infected with a wild-type BHV5 strain or vaccinated against BHV5 from within a population of ruminants vaccinated with a vaccine according to claim 21 comprising testing a ruminant for the presence or absence of an antibody directed against the glycoprotein I (gI) gene or the glycoprotein E (gE) gene or both.

23. A test kit provided with means for performing a method according to claim 22.
